# EUROPEAN PATENT SPECIFICATION

(11) **EP 4 126 140 B1**
(45) Date of publication and mention of the grant of the patent: **17.12.2025**
(21) Application number: 21724730.3
(22) Date of filing: 06.04.2021
(51) Int. Cl.: A61M 15/00, G16H 20/13, G16H 50/20, A61B 5/00, A61B 5/08

(54) **INHALER SYSTEMS**
INHALATORSYSTEME
SYSTÈMES D'INHALATEUR

(30) Priority: 04.04.2020 GB 202005000
(43) Date of publication of application: 08.02.2023
(73) Proprietor: Purcell Global Ltd, London, City of London WC2H 9JQ (GB)
(72) Inventor: PURCELL, Nicholas, London City of London SW16 3EP (GB)
(74) Representative: Valet Patent Services Limited
(86) International application number: PCT/GB2021/050847
(87) International publication number: WO 2021/198712

(56) References cited:
- WO-A1-2012/072249
- WO-A1-98/41253
- WO-A2-2014/204511
- WO-A2-93/12823
- GB-A- 2 447 606
- GB-A- 2 450 327
- GB-A- 2 552 720
- GB-A- 2 565 797
- US-A1- 2019 385 727

## Description

### Technical field

The present invention relates to inhaler devices, such as metered dose inhaler devices, dry powder inhaler devices and soft mist inhaler devices. The present invention relates particularly, but not exclusively, to inhaler devices which comprise an electronic component permitting communication with an external client device. Such inhaler devices are sometimes referred to as "smart inhalers", and can be used within an inhaler system comprising an inhaler device and an external client device in communication with the inhaler device.

### Background

An inhaler device (or "inhaler") is a medical device used for delivering a powder or a liquid (in the form of an aerosol or mist), usually medication, into the body via the lungs. Such devices are mainly used in the treatment of respiratory diseases, such as asthma and chronic obstructive pulmonary disease (COPD). However, inhaler devices may also be used for delivery of any medication which may be inhaled, e.g. aerosolised, such as pain relief, medical marijuana, breath fresheners, etc.

Inhaler devices typically comprise a housing for receiving a vessel containing one or more doses of the substance to be delivered. In the case of a metered dose inhaler, such a vessel is typically a canister operable to deliver a metered dose of a substance (e.g. a medicament) held inside the canister. In a dry powder inhaler such a vessel may comprise a blister including a single dose of a substance in powder form or a reservoir including multiple doses of such a substance. In a soft mist inhaler such a vessel may comprise a cartridge including multiple doses of the substance to be delivered in liquid form. In each case, the housing usually comprises a mouthpiece. In use, a user of the inhaler device may trigger the inhaler to deliver a dose of the susbstance, for example by pressing a button on the housing, by pressing on the canister itself (in the case of a metered dose inhaler), by turning the cartridge (in the case of a soft mist inhaler). The user may then inhale an aerosolised dose of the substance / medication via the mouthpiece. Some inhalers may be triggered to deliver a dose by the act of the user inhaling (such inhalers may be termed breath actuated inhalers).

There can be problems associated with typical inhaler devices, one of which is an inability for the user to know when the device will require replacement or refilling. It can also be important for a user of an inhaler device to keep to a defined dosage regime. This can be difficult using many known inhaler devices unless the user makes use of a separate reminder schedule. Furthermore, it can often be helpful for a medical professional associated with a user to know details of when and how the user has used their inhaler device, in order to improve on future treatment. Again, this information can be difficult and/or impossible to obtain using typical inhaler devices, unless the user maintains a record of their usage.

Smart inhaler devices have been proposed which are able to monitor inhaler usage. However, these devices can still suffer from poor user uptake. Some users may perceive there to be a social stigma associated with use of an inhaler device, and therefore may be reluctant to use the device as recommended by their medical practitioner. Many such devices also suffer from poor ergonomics and incorrect usage.

Some prior art inhaler devices are known from the documents GB 2 552 720 A, WO 2012/072249 A1, WO 2014/204511 A2 and US 2019/385727 A1.

It is an object of the invention to provide an improved inhaler device and system which alleviates some of the above problems.

### Summary of the invention

The present invention provides an inhaler device as defined in claim 1, and an inhaler system as defined in claim 13. Further preferred embodiments of the present invention are defined in the dependent claims.

### Summary of the disclosure

Reference will now be made to various exemplary embodiments or aspects of the present disclosure. Embodiments or aspects disclosed herein which do not fall under the scope of the appended claims do not form part of the present invention, but are useful for understanding the principles of the invention. The scope of the present invention is defined by the appended claims.

According to a first aspect of the disclosure we provide an inhaler device operable to deliver a dose of an active ingredient, the inhaler device comprising: a housing comprising a mouthpiece; a removable flow restrictor at least partly received within the housing, the flow restrictor defining a cavity operable to receive a vessel comprising the active ingredient; and an electronic component operable to monitor inhaler data and to transmit the inhaler data to a remote device.

Such an inhaler device is conveniently reusable and may be used as part of an inhaler system, as described later.

The flow restrictor may be a filter. The vessel may comprise a cartridge, reservoir, canister, blister or similar, depending on the type of inhaler device.

The electronic component may be located within the removable filter such that together the removable filter and the electronic component form a removable core. The removable core allows the inhaler device to be easily disassembled and cleaned.

The electronic component may comprise a power source, such as a battery, solar cell or kinetic charger. This avoids the need for the device to be connected to an external power source in order to function. Nevertheless, the inhaler device may be connected to an external power source and/or the battery may be rechargeable via a cable or wirelessly.

The inhaler may further comprise a cap operable to cover the mouthpiece. The cap may be secured to the filter and/or the housing using a magnetic closure. If required, the electronic component may be included in the cap of the inhaler device, or the electronic component may be partly provided within the inhaler device and partly provided in the cap. The cap maintains the inhaler device in a sanitary condition, and the magnetic closure permits easy removal and replacement of the cap.

The inhaler data may comprise one or more of: usage data, location data and dosage data.

The inhaler may comprise a dosage counter operable to count or otherwise determine dosage data, such as a number of doses administered by the inhaler. The electronic component may be operable to transmit an alert related to the number of doses delivered, for example when the number of doses administered exceeds a predefined threshold, or when a number of dose administered does not meet a minimum threshold.

The inhaler may comprise a location sensor, such as a GPS receiver operable to determine location data such as a current location of the inhaler. The electronic component may be operable to transmit location data.

The inhaler may comprise one or more sensors operable to determine usage data. Such sensors may include, but are not limited to, one or more microphones, image sensors, air flow meters, accelerometer, magnetometers.

The inhaler may further comprise an indicator component. The indicator component may be an indicator ring connectable between the housing and the removable filter. The removable filter may be shaped to regulate the speed of a fluid flowing though the inhaler. For example, the surface area may be shaped to regulate the fluid flow. In one example the removable filter comprises one or more channels or openings operable, e.g. shaped, to regulate a speed of a fluid flowing through the inhaler.

The housing may define a body portion having a first longitudinal axis and a mouthpiece portion having a second longitudinal axis may be oriented at an angle to the first longitudinal axis, wherein the angle is preferably in the range 125-140 degrees, for example 130-135 degrees, and most preferably 133 degrees. It will be appreciated that the housing angle may be selected to reflect and comply with an existing medication inhaler(s) geometries.

The housing may comprise a translucent portion or may be substantially translucent. The housing may be opaque, e.g. metal.

An outer surface of the filter may substantially abut an inner wall of the housing, such that when a vessel such as a canister is inserted into the cavity within the filter the filter substantially fills a space between the vessel and the inner wall of the housing.

According to a second aspect of the disclosure we provide a cap operable to cover a mouthpiece of an inhaler device, the cap comprising an electronic component operable to monitor inhaler data and to transmit the inhaler data to a remote device. The inhaler data may comprise one or more of: patient data, usage data, location data and dosage data.

The cap may be provided in combination with an inhaler device that is operable to deliver a dose of an inhalable active ingredient, the inhaler device comprising a housing comprising a mouthpiece and a removable flow regulator at least partly received within the housing, the flow regulator defining a cavity operable to receive a vessel comprising the active ingredient. In particular, the cap may be provided in combination or may be configured for use with an inhaler device in accordance with the first aspect of the disclosure.

The cap could alternatively be provided as a "stand alone" device that could be fitted to an existing inhaler device.

According to a third aspect of the disclosure we provide an inhaler system, the system comprising: an inhaler according to the first aspect of the disclosure and/or a cap according to the second aspect of the disclosure, and a computer programme application operable, when run on a client device, to cause the client device to receive inhaler data from the electronic component.

The computer programme application may be further operable to receive supplementary data from a data source other than the electronic component, wherein the supplementary data comprises at least one of: user data, sensor data, and environment data.

The computer programme application may be further operable to transmit the received inhaler data and supplementary data to a remote server over a network. The transmitted data may be stored in a cloud-based storage system and may be accessed by a user from the client device or from another device in communication with the network.

The computer programme application may be further operable to store the received inhaler data and supplementary data in a memory of the client device.

The inhaler data may comprise location data indicating a physical location of the inhaler device and/or cap, and the computer programme application is further operable to display to a user of the client device a current location of the inhaler device. The inhaler data may comprise usage data indicating one or more times at which a dose was administered using the inhaler device or that zero doses have been administered by the inhaler device, and the computer programme application is further operable to display the usage data to a user of the client device. The inhaler data may comprise dosage data indicating a number of doses administered using the inhaler device, and the computer programme application is further operable to display the dosage data to a user of the client device.

The computer programme application may be in communication with network, and the computer programme application may be operable to search for and retrieve environment data using the network.

The computer programme application may be in communication with a network, and the computer programme application may be operable to receive anonymised inhaler data and/or anonymised supplementary data from other users of the system.

The features of the third aspect of the disclosure may be combined with the features of the first and second aspects of the disclosure in any order, as well as with features selected from the description.

### Brief description of the drawings

Embodiments of the disclosure will now be described, by way of example only, with reference to the accompanying drawings, in which:
**Figure 1** shows a schematic diagram of an inhaler device within an inhaler system;
**Figure 2** shows a side view of an inhaler device;
**Figure 3** shows a front view of the inhaler device of Figure 2;
**Figure 4** shows a top view of the inhaler device of Figure 2;
**Figure 5** shows an exploded side view of the inhaler device of Figure 2, with some internal features visible in broken lines;
**Figure 6** shows a cross section through the inhaler device of Figure 2;
**Figure 7** shows an exploded perspective view of the inhaler device of Figure 2;
**Figure 8** shows an exploded front view of the inhaler device of Figure 2; and
**Figure 9** schematically illustrates airflow through the inhaler device of Figure 2.

### Detailed description

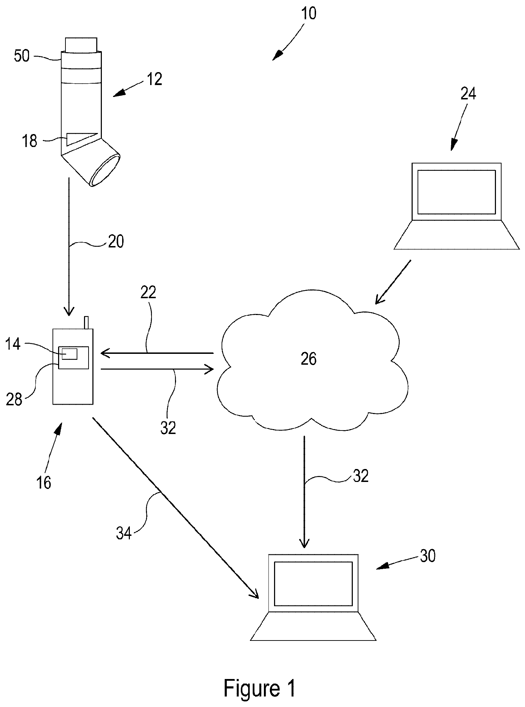

Referring first to Figure 1, and inhaler system is shown, which in this case is a metered dose inhaler system 10. The system includes an inhaler 12 and a computer programme application 14, which is operable to be installed on a client device 16 that is remote from the inhaler 12. In the example shown in Figure 1 the client device 16 is a mobile phone, which may belong to a user of the inhaler 12.

The inhaler 12 depicted in Figure 1 is a metered dose inhaler, but it will be appreciated that the inhaler could alternatively be another type of inhaler device, such as a dry powder inhaler or soft mist inhaler. The inhaler 12 is a smart inhaler device, in that the inhaler includes an electronic component 18, which is operable to monitor inhaler data and to transmit that inhaler data to a remote device, for example the client device 16, as indicated in Figure 1 by arrow 20.

The computer programme application 14 is operable, when run on the client device 16, to cause the client device 16 to receive the inhaler data from the electronic component 18. The computer programme application 14 is further operable to cause the client device 16 to receive supplementary data from a data source other than the electronic component 18, as indicated by arrow 22. Such a data source may, for example, be a remote computer or server 24 with which the computer programme application 14 is in communication via a network 26, such as the internet. Alternatively, or additionally, the data source may be another component of the client device, such as an internal memory 28 of the client device or a sensor comprised within or available to the client device, such as an accelerometer or heart rate monitor.

The computer programme application 14 may further be operable to transmit the received inhaler data and/or supplementary data to a remote server 30. Such transmission may be over the network 26, as indicated by arrows 32, or might be via a direct wired or wireless link, as indicated by arrow 34. The transmitted data may be securely stored on a secure cloud platform for access, for example, by the authorised user of the device and/or healthcare professional. Alternatively or additionally the computer programme application may be operable to store the received inhaler data and supplementary data in the memory 28 of the client device.

The core purpose of the system 10 illustrated in Figure 1 is to provide a device and technology platform to help patients, care givers, general practices, government bodies, and the pharmaceutical Industry improve quality of life for users of inhaler devices, such as those suffering from asthma and COPD or patients requiring other inhaled medications / liquids.

As noted above, the system 10 is operable to record inhaler data, such as usage, location, and dosage data, together with supplemental data, such as patient information and environmental factors, in order to aid in improved disease management. This data may be provided to the user (e.g. displayed to the user on the client device 16 via the computer programme application 14) to allow the user to better understand the triggers associated with their condition. The data may also be provided to a healthcare practitioner associated with the user in order to help that practitioner to improve the treatment options available to the user. The collected data (suitably anonymised) may also be used to better inform Government and Industry bodies, for instance to assist them in planning for and managing people affected by a certain condition, to enable better insight around hot spots (e.g. locations/time periods of poor air quality and/or high pollen count), and to better understand user behaviour. All data collected could be cloud based if required, thus allowing access to the collected data by any entity having appropriate permission.

Examples of usage data may include, but are not limited to, inhalation speed, inhalation sound, one or more images of the user's throat or mouth, time of dose delivery. To this end the inhaler may comprise an airflow meter operable to record one or more of an inhalation/exhalation maximum speed, average speed, and duration. Alternatively or additionally the inhaler may include a microphone operable to record or otherwise monitor sound produced when the user inhales and/or exhales, to assist in gauging respiratory function. The inhaler may alternatively or additionally include a camera or other imagining sensor (an example being the Sonicare FlexCare Platinum) to image a portion of the users mouth and/or throat.

Examples of location data may include but are not limited to a location at which a dose was delivered, a current location of the inhaler device, a last known location of the inhaler device. To this end the inhaler, e.g. the electronic component, may include a location sensor such as a GPS sensor.

Examples of dosage data may include but are not limited to number of doses delivered by the inhaler device and/or number of doses remaining in the inhaler device. To this end the inhaler may include a dose counter.

A primary purpose of the system 10 is, as discussed above, to improve disease management by using smart technology and a personalised app to record inhaler dosage, usage, location, environment and other data in order to inform the patient (and/or other parties) and to allow them to review their treatment.

However, a secondary purpose of the system 10 is to encourage user compliance with a treatment regime. Many common inhaler devices are disposable. They are thus often constructed from low cost materials, and are unattractive and/or difficult to use correctly. In order to improve the user experience the applicant has found it desirable to improve the perceived quality and value of an inhaler device, so encouraging the user to keep their inhaler device close to them at all times. It is furthermore desirable to make the inhaler device more appealing, pleasant and efficient to use.

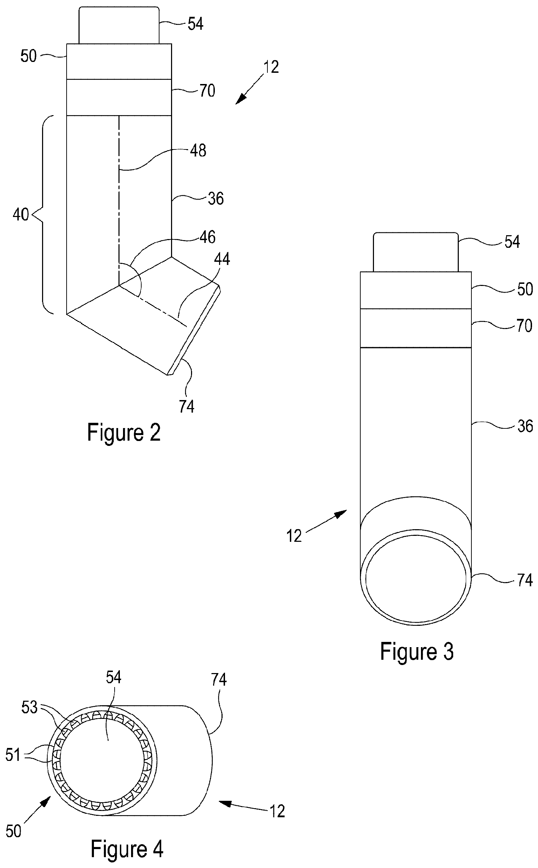

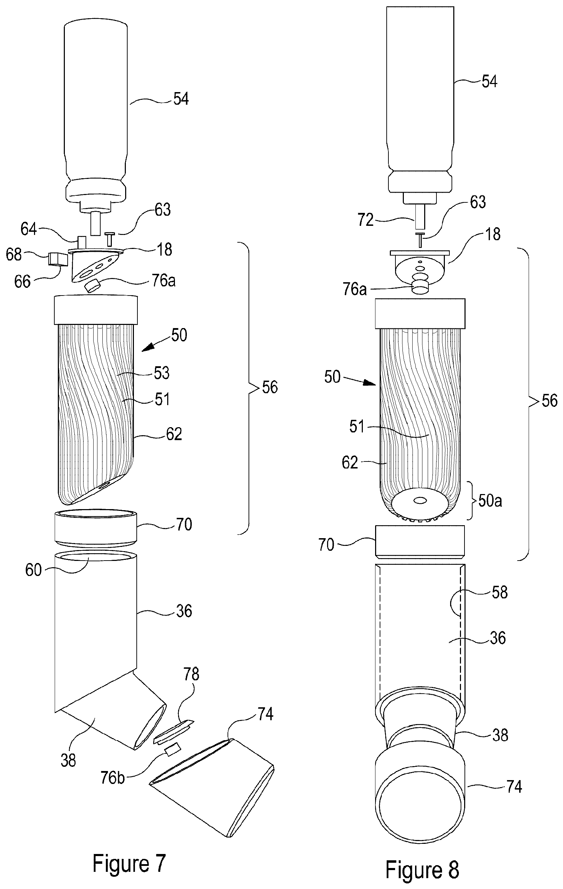

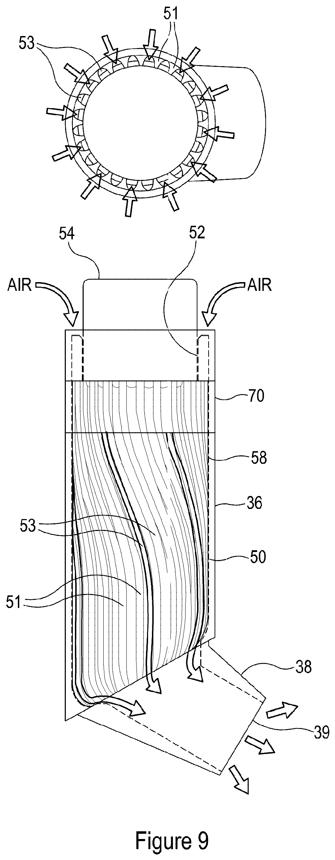

Designing an inhaler with a longer lifespan than a disposable inhaler device has created a number of challenges. A reusable device must be able to be easily cleaned, and to ergonomically incorporate the smart electronic component needed to track inhaler dosage and usage. Such a reusable inhaler device 12 is shown in Figures 2 to 9. Although Figures 2 to 9 show a metered dose inhaler it will be appreciated that the principles discussed below are equally applicable to other types of inhaler, such as dry powder inhalers and soft mist inhalers.

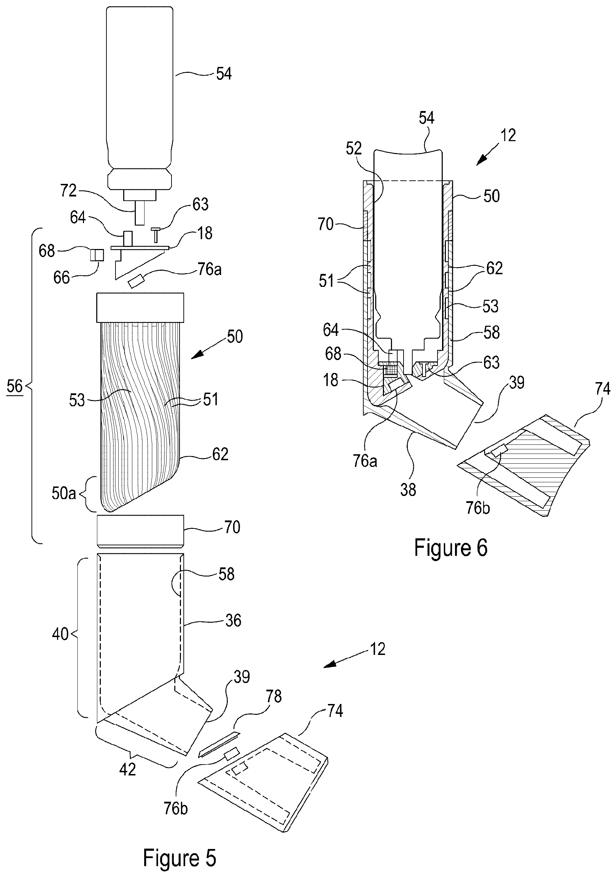

Figures 2, 3 and 4 respectively show side, front and top views of a metered dose inhaler device 12 operable to deliver a metered dose of an aerosolised active ingredient, usually a liquid. Figures 5, 7 and 8 respectively show side, perspective and front exploded views of the inhaler device 12, whilst Figure 6 shows a cross-section through the inhaler device 12. The inhaler device 12 includes a housing 36, which has a mouthpiece 38 defining an opening 39 through which a user of the device may inhale the aerosolised active ingredient when the device is in use.

In the example shown the mouthpiece 38 is integral with the housing 36. The housing 36 thus includes a body portion 40 and a mouthpiece portion 42. Each of those portions comprises a respective longitudinal axis. The longitudinal axis 44 of the mouthpiece portion 42 is at an angle 46 to the longitudinal axis 48 of the body portion 40. The angle 48 is, in the example shown, approximately 133 degrees, but in other examples the angle may be in the range 125-140 degrees, for example in the range 130-135 degrees. This range of angles, centring on 133 degrees, was selected to give more space for the user's nose than is typically provided in disposable inhalers, and to provide a comfortable grip when using the inhaler device. It will be appreciated however that this angle may alternatively be selected to reflect or comply with existing inhaler geometries. Both the body portion 40 and the mouthpiece portion 42 are substantially cylindrical, to provide a compact shape to the inhaler device. It will nevertheless be appreciated that the device could have other cross sections or shapes if required.

The mouthpiece may have an opening that is wider than a typical inhaler opening. Typically inhaler openings have a circular circumference. In contrast, the device shown in Figures 2 to 9 has an opening having a circumference that is flattened at its upper and lower extremities, so as to give a generally elliptical shape. This results in a mouthpiece that has a more organic shape and feels more natural to use. In the example shown the mouthpiece opening has a widest dimension along a horizontal axis of approximately 22mm, with a radius of approximately 9mm, whilst the widest dimension in the vertical direction (perpendicular to the horizontal direction) is less than that of the vertical dimension, for example 90, 85 or 80 percent of the horizontal dimension (in this case approximately 18mm).

The mouthpiece cross section may change along its length. For example, the mouthpiece may be shaped to taper towards the opening. In the example shown in the Figures the mouthpiece has a substantially circular cross section where it abuts the housing (having a diameter of approximately 26mm). The cross section tapers and flattens towards the mouthpiece opening. Of course, if required a standard circular cross section could be used instead.

The inhaler device 12 further includes a removable flow regulator 50, also termed herein a removable filter. The removable flow regulator 50 is at least partly received within the housing 36. The removable flow regulator 50 defines a cavity 52 that is operable to receive a vessel comprising a substance to be delivered to the use. As the inhaler shown in Figures 2 to 9 is a metered dose inhaler, the vessel is in this example a canister 54 comprising an active ingredient to be aerosolised. "Removable" as used herein means that the inhaler device may be disassembled by removing the flow regulator and subsequently reassembled by replacing the same flow regulator (or another similar flow regulator).

In the example shown the housing has an inner wall 58 that defines an interior space 60. An outer surface 62 of the flow regulator 50 is shaped to have a complementary shape to the inner wall 58, such that when the flow regulator 50 is inserted into the interior space 60 of the housing 36 the outer surface 62 of the flow regulator substantially abuts the inner wall 58 of the housing. This ensures that when a canister 54 is inserted into the cavity 52 within the flow regulator 50, there is very little free space between the canister 54 and the inner wall 58 of the housing. Like the body portion of the housing, the flow regulator 50 is substantially cylindrical in shape. However, it will be appreciated that the flow regulator could have other shapes if required, in order to cooperate with the available internal space within the inhaler into which it is to fit.

In typical inhaler devices, there is often a large gap between the medicament containing vessel (e.g. aerosol canister) and housing, which allows dust and dirt to collect in the space around the canister. This debris is then often inhaled by a user when using the device, creating a potential hazard, causing discomfort and loss of confidence in the device. The removable flow regulator 50 can prevent such debris from accumulating, and may also operate to filter inhaled air to remove particles, such as particles picked up from pockets and bags (hence why it is also termed herein a "removable filter"). Because the filter 50 is removable it may be easily cleaned to remove the filtered particles.

The flow regulator / filter 50 may also regulate the speed of air drawn through the inhaler when in use. In the example shown, the flow regulator comprises a plurality of ribs 51 which define air flow channels 53 between pairs of adjacent ribs 51. The ribs are each curved into an elongated S shape. The channels 53 have a substantially U-shaped cross section. The filter regulates the airflow through the device by limiting the space between the ribs in order to control the speed at which air may be drawn through the filter. The shape of the ribs increases the length of the air flow path through the filter. Regulating the air flow in this manner may encourage the user to extend the length of their inhale, giving more time to dispense the medicine into the lungs correctly. The U shaped cross-section of the channels ensures that the filter may be easily cleaned when it is removed from the housing.

It will be appreciated that other filter designs are possible which would also achieve the function of regulating air flow through the inhaler. For example, the air-flow channels could define a different path shape to that shown, which nevertheless may regulate, e.g. limit the speed at which air may flow through the device. Similarly, the channels may have a different cross section. Alternatively, or additionally, air flow may be controlled via constrictions or openings having a size selected to regulate, e.g. limit, flow speed. The air flow may also be controlled be careful design of the outer surface of the filter, for example by provision of dimples, like those on a golf ball. Air flow could be controlled in conjunction with or independently of the mouthpiece orifice shape and dimension and/or the internal geometries of the actuator body. As a further alternative, the flow regulating features (e.g. channels, dimples, etc) could be provided on the housing rather than on the flow regulator, or may be provided partly on the housing and partly on the flow regulator.

As explained above, the inhaler 12 includes an electronic component 18 that is operable to monitor inhaler data and to transmit the inhaler data to a remote client device. In the example shown, the electronic component 18 is located within the removable filter 50 such that together the removable filter 50 and the electronic component 18 form a removable core 56. The core 56 may thus be removed from the inhaler to disassemble the device and replaced to reassemble the device at a subsequent time. Providing the electronic component within such a removable core 56 allows for effective cleaning of the housing 36 (including mouthpiece) as well as effective cleaning of the filter 50. It also allows for easy assembly and maintenance of the electronic element 18. For example, the electronic element may be removably secured within the filter 50 using a removable fastener 63, such as a screw. Nevertheless, it will be appreciated that the electronic component could be included in a different part of the inhaler device, separate from the removable filter, if required. For example, the electronic component may be provided in the cap. The electronics may be distributed between two (or more) electronic components which may be located in different parts of the device, e.g. one electronic component within the filter and one within the cap. Wherever it is (or they are) located, the electronic component(s) may be housed within a water resistant or waterproof compartment.

To accommodate the electronic component 18, in the example shown, the filter 50 includes an extended portion 50a below the portion of the filter which houses the canister. This gives the filter 50 the shape of a chamfered cylinder so that the filter fits closely within the body portion 40 of the housing 36. The electronic component may be housed within the extended portion of the filter, below the canister. For example, the cavity 52 which holds the canister 54 may extend into the extended portion 50a such that both the canister 54 and the electronic component are accommodated within a single cavity 52.

The electronic element 18 may include a power source (not shown) such that the device is self powered, and does not require connection to an external power source or charger. The power source may be a kinetic charger. Such a charger converts kinetic energy into electrical power, and thus encourages the user to shake the device prior to use, which is also necessary to emulsify the propellant in the aerosol canister. An alternative power source may be used instead if preferred, such as a battery or solar charger. The battery may be rechargeable, and charged via an external power source.

The electronic component 18 may be operable to collect inhaler data. Such data may comprise usage data, which may be for instance data indicating the time at which the inhaler was activated to deliver a dose of the active ingredient. Such data may also or alternatively comprise dosage data, which may indicate a number of doses that have been delivered by the device. Such data may also or alternatively comprise location data, which may indicate a location of the inhaler device, for example using GPS coordinates.

To assist in the collection of the inhaler data, the electronic component may include an internal processor having a timing clock. The processor may be in communication with a dosage counter 64, such as a pressure switch or a piezoelectric switch, and/or a GPS receiver 66.

One or more usage sensors (not shown) may be included in the inhaler if required, such as a microphone, image sensor, air flow sensor, particle sensor, gyroscope, accelerometer and magnetometer. These sensors, if present, may be in communication with the processor and may be operable to collect data (e.g. data indicative of the sound of a user's inhalation/exhalation, one or more images of an interior of the user's mouth or throat, data relating to the speed of an inhalation) and deliver that data to the processor. The usage data recorded by the one or more usage sensors may be used to listen to or calculate the flow rate and length of inhalation. Recorded peak inspiratory flow rates (PIFR) or Peak Flow readings can be used to base line this. The microphone may also be used to listen to the lung function / health.

The electronic component may also include a transmitter/receiver 68, such as a Bluetooth (TM) transmitter/receiver or other near field transceiver, using which the electronic component may transmit the collected inhaler data to the application 14 installed on the client device 16.

The inhaler device 12 shown in Figures 2 to 9 further includes an indicator component 70. The indicator component 70 is, in the present example, in the form of a ring that is connectable between the filter 50 and the housing 36, for example by frictionally engaging both the housing and the filter. such as via a push fit or a screw fit. Such an indicator component 70 may be used to provide an indication of the drug contained within the canister 54. For example, the name of the drug may be written on the indicator component. The indicator component may also be colour coded to provide an indication of the drug held within the device from a distance and/or may comprise an indication of the drug in braille for the partially sighted. The indicator component helps to prevent confusion in cases where a user may have multiple inhaler devices. In the example that is shown in the drawings, the inhaler device will not assemble correctly without the indicator component (because the filter cannot be secured to the housing without it), thus forcing the user to make use of the indicator component. Nevertheless, an indicator component need not be included in other inhaler designs, if not required.

The inhaler device 12 of this specific example, and in particular the cavity 52 within the filter 50, is shaped to accept a standard commercially available drug-delivery canister. Such canisters typically include an internal metering valve and an actuator, and are designed such that when the actuator is activated (usually by pressure) a metered dose of an active ingredient contained within the canister is aerosolised.

In order to use the inhaler device 12, a user must first shake the device to emulsify the propellant within the canister. This advantageously simultaneously kinetically charges the electronic component. The user must then press down on the canister 54 in order to activate the canister actuator 72, and so deliver a metered dose of the active ingredient within the canister. This advantageously simultaneously activates the dosage counter 64.

It will be appreciated that in other examples, the cavity 52 within the flow regulator / filter 50 may be shaped to receive a different type of vessel, which may also be a standard commercially available medicament vessel, such as a dry powder blister / blister pack or reservoir, or a soft mist cartridge. The outer dimensions of such removable filters (i.e. the shape of the outer surface 62 that is complementary to the shape of the inner wall 58 of the housing, such that when the filter 50 is inserted into the interior space 60 of the housing 36 the outer surface 62 of the flow regulator substantially abuts the inner wall 58 of the housing) may be the same, so as to define a standard size, such multiple different types of filter may be used within the same inhaler housing.

The inhaler device 12 further includes a cap 74 to protect the mouthpiece when the inhaler device is not in use. The cap comprises a closure 76, which in the example shown is a magnetic closure. A first portion 76a of the magnetic closure is provided within the housing 36 when the device is assembled, for example within the removable core 56. A second portion 76b of the magnetic closure is provided within the cap 74, for example beneath a cap top 78. Provision of a cap 74 with a magnetic fastening 76 enables fast removal in an emergency and a more pleasant and satisfying experience during general use. Additionally regular caps are frequently lost which we aim to minimize. The cap may also or alternatively include some or all of the electronic elements. For example, the cap may be "strapped" to an inhaler such that when the cap is removed it is still "fixed" to the inhaler device. A pressure or haptic sensitive dosage counter could be in, or could be, the strap connecting the cap with the inhaler. Such a strap may be, for example, located around the base of the inhaler where the user's thumb or finger typically sits. As described above, the dosage sensor communicates with the electronics in the cap, which are operable to send data (e.g. dosage data) to the client device.

In an arrangement having a magnetic closure, the magnetic closure could also act as a connection for data and/or power transfer between electronic components in the inhaler body (e.g. the filter) and the cap. For example, the cap may comprise a charging connector, and the magnetic closure may transfer power from the cap to a battery located in the filter to charge the battery in the filter. Inhaler data gathered by the filter could be transferred via a data link in the magnetic closure to the cap (and then to the client device), or vice versa.

The inhaler may include an inhalation velocity indicator, operable to indicate to the users that they are inhaling through the device at an appropriate speed (e.g. fast enough). Such an indicator may be mechanical (e.g. movable element operable to indicate a flow in proportion to a displacement distance), digital (e.g. informed by an airflow sensor, as discussed above) or auditory (e.g. a sound produced when a velocity threshold is reached or exceeded - such a sound may result from the shape of the filter, e.g. the shape of the air flow channels).

Similarly, the inhaler may include an activation or priming indicator, to confirm to a user that a dose of medicament has been correctly activated. For example, a mechanical, physical, digital or audible indication may be produced when a canister has been pressed down correctly so as to deliver a dose.

The inhaler may include one or more light sources, e.g. one or more LEDs. The light source(s) may assist with locating the device (e.g. the electronic component may be caused to operate the light source in response to an instruction from the application on the client device). Further the light source(s) may be illuminated to indicate to the user information regarding usage / dosage / inhalation / technique / battery etc.

The inhaler may include luminescent material, e.g. a luminescent coating. Again this may aid with locating the device, and may be particularly useful for sight impaired patients and/or for engaging children with the device.

It will be appreciated that the inhaler device 12 functions in a similar manner to a standard (i.e. non-smart) inhaler device when it is not in communication with the inhaler system 10. In order to use the inhaler device 12 within the inhaler system 10, a user first downloads the inhaler computer programme application 14 to a client device 16, such as their mobile phone, tablet device or personal computer. The user then pairs their inhaler device 12 with the application 14 installed on their client device. This may be done using a known handshake-type pairing method, such as that used by the Bluetooth (TM) standard. In particular, the client device may transmit a connection request to the electronic component of the inhaler device, which may transmit a reply accepting the connection request (or vice versa). Once paired a user may need to actively de-sync the device from their client device. This is to make the inhaler device secure and if lost traceable.

Once paired, the electronic component 18 of the inhaler device 12 is able to transmit inhaler data to the client device 16 and receive data from the client device. Such data may be transmitted at regular intervals and/or after a usage event. Data may be transmitted only when the inhaler device 12 is within range of the client device 16 and may, for example, be transmitted in response to a request from the client device. The electronic component may thus include an internal memory in which to store inhaler data until the data can be transmitted. Received inhaler data may be stored in one or more records in a memory of the client device.

The computer programme application 14 may be operable to search for supplementary data, such as environmental data, which might include data relating to air quality (e.g. an air quality index), weather, pollen count or another environmental factor which could be linked to a worsening of the patient's condition. The environmental data may be stored in a memory of the client device in association with the stored inhaler data. For example, data relating to a specific usage event (e.g. sensor data, time, dosage count, and/or location) may be stored in a record together with data relating to environmental conditions at the time of the event.

The application may be operable to alert the user when environmental conditions may cause their condition to worsen (e.g. when the air quality drops below a threshold figure, and/or when pollen count rises above a threshold figure). This may remind the user to take their inhaler device with them.

The application may be in communication with external services such as electronic prescription services, doctors' practices (e.g. the user's registered doctor) and/or hospitals (e.g. a nearest emergency department) and emergency services. The application can be operable to determine from the received usage data that the patient is experiencing severe difficulty breathing (e.g. having severe asthma attack). This could be by comparing, using e.g. an AI or machine learning, received usage data to historical usage data (either from the patient or collected from other patients). Once it has determined that a severe attack is occurring the application may be operable to prompt calling emergency services (or to automatically call emergency service. Data collected by the app, such as inhaler data and environment data, may be provided to the emergency services. This may assist an emergency practitioner in locating the patient and/or in diagnosing the patient's condition.

The application may be operable to alert the user when a number of doses dispensed by the inhaler device rises above a threshold figure. This may trigger the user to replace the drug delivery canister. Similarly, the application may be operable to alert the user when a number of doses dispensed by the inhaler device does not meet a minimum threshold figure. This may trigger the user to utilise their inhaler.

The application may be operable to request inhaler data from the electronic component at the request of a user. For example, the user may request the application to request location information from the inhaler device. The application may then display the requested location information to the user on a map to allow the user to locate their device(s). Similarly, the user may request the application to request usage information from the inhaler device. The application may then display to the user information obtained from the device sensors, such as inhalation speed, to assist the user in improving their inhaler technique.

The application may also be configured to permit a user to define alarms or notifications if movement of the client device is detected and the inhaler is not close or present. This helps to remind the user to take the inhaler with them.

The user may additionally input personal data into the application, such as information about their condition. The application may be operable to obtain other user data via the client device, for example data relating to user activity, such as step count, heart rate, etc.

The application may comprise software operable to display a treatment regime to the user. Gamification may encourage the user to comply with the treatment regime (e.g. rewards may be provided for compliance). The application may further provide guidance on lung function training, and may utilise sensor data recorded from the inhaler to gauge user lung function and/or change in lung function.

All the data received by the application may, if required, be transmitted to a remote server, such as a computer associated with a medical professional or research institute. The data may alternatively or additionally be stored on a secure "Cloud" platform accessible by the user and/or other entities from alternative computed devices accessible to the internet.

The inhaler system 10 described herein thus provides a data log linking three core pieces of data:
A = Information about the Individual; Treatments, Dosage counts, heart rate, etc.
B = Information about their Environment; the current location or plan to travel / be in: Weather, pollen counts, pollution
C = Information about their condition / treatment / medication;

Such data enables an individual to better understand their condition and its triggers. It also allows healthcare professionals to enhance the treatment of the patient's disease, for example by permitting easy review of the patient's treatment. The system may be operable to permit the healthcare practitioner to provide treatment alterations or recommendations to the patient via the app.

User data from a plurality of users may be collected by the system, anonymised, and used to assist researchers and medical professionals in understanding effectiveness of treatment regimes. Such usage analytics may be helpful in sharing experiences across the user population. For instance, anonymised usage data may be provided to the client device for access by the user of the device. The "desensitized" usage data may be provided together with location data, so allowing information regarding other users of the system to help a user in the same location as those other users. Similarly, the desensitized data may be provided together with anonymised user data, such as details of user activities, like exercise, and details of user conditions. This may allow a user with a specific condition to see how a selected activity, such as a type of exercise, has affected other users with the same or a similar condition to their own.

Any data collected by the device or from supplementary sources could be utilised to build data models for things like "attack prediction" / for better disease insights. This could be done with data science tools, for example Artificial Intelligent or Machine Learning. This data could be anonymized and or aggregated for patient/disease comparisons and deeper learning and modelling for research, e.g. for monitoring health metrics like BPM and Sp02 levels pre/post/during an attack and analysing any multiple of other data like environmental AQI's (air quality index).

An inhaler device of the type described herein is designed for a long life, as it can easily be disassembled for efficient cleaning. If required, the components of the device may be selected such that the device components are fully recyclable. The device may be made using a material pallet associated with high end personal devices and accessories such as smart phones and earphones, in order to potentially alter the patient's perception of the inhaler. For example, a device which is considered a cherished personal accessory will be more likely to be carried on the person more often and the user will be less inhibited to use it in public. Personalisation may be through colour or material choice or images. Examples of materials that might be used for the housing include metal (e.g. aluminium) resin, plastic, PCR plastics and bioplastics or acetate. The housing may be provided in a range of user-selectable colours and/or with user-customisable printing. The filter may be made from aluminium or other materials as described for the housing. Of course, the device may be made from other materials, including lower end materials, if preferred, such as those used for existing inhaler devices.

## Claims

1. An inhaler device (12) operable to deliver a dose of an inhalable active ingredient, the inhaler device comprising:
a housing (36) comprising a mouthpiece (38);
a removable flow regulator (50) at least partly received within the housing, the flow regulator defining a cavity (52) operable to receive a vessel (54) comprising the active ingredient; and **characterised by**
an electronic component (18) operable to monitor inhaler data and to transmit the inhaler data to a remote device (24, 30), wherein the electronic component is located with the removable flow regulator such that together the removable flow regulator and the electronic component form a removable core (56).

2. The inhaler device of claim 1, wherein the electronic component comprises a power source.

3. The inhaler device of any preceding claim, wherein the removable flow regulator is a removable filter.

4. The inhaler device of any preceding claim, wherein the inhaler device further comprises a cap (74) operable to cover the mouthpiece, wherein the cap is optionally secured to the housing and/or the flow regulator using a magnetic closure and wherein data and/or power is optionally transferred to or from the electronic component via the magnetic closure.

5. The inhaler device of any preceding claim, wherein the inhaler data comprises one or more of: usage data, location data and dosage data.

6. The inhaler device of any preceding claim, wherein the inhaler device comprises a dosage counter (64) operable to count a number of doses administered by the inhaler device, optionally wherein the electronic component is operable to transmit an alert when the number of doses administered exceeds a predefined threshold or fails to meet a defined number.

7. The inhaler device of any preceding claim, further comprising an indicator component (70), wherein the indicator component is optionally an indicator ring connectable between the housing and the removable flow regulator, and/or wherein the removable flow regulator comprises a structure (51, 53) operable to regulate a speed of a fluid flowing through the inhaler device, wherein the structure optionally or preferably includes one or more channels, dimples or openings.

8. The inhaler device of any preceding claim, wherein the housing defines a body portion (40) having a first longitudinal axis and a mouthpiece portion (42) having a second longitudinal axis is oriented at an angle to the first longitudinal axis, wherein the angle is in the range 130-135 degrees, and/or wherein:
(i) the housing comprises a translucent portion or is substantially translucent; and/or
(ii) the housing comprises a metal portion and/or an opaque coloured portion.

9. The inhaler device of any preceding claim, wherein an outer surface (62) of the flow regulator substantially abuts an inner wall (58) of the housing, such that when the vessel is inserted into the cavity within the flow regulator the flow regulator substantially fills a space between the vessel and the inner wall of the housing.

10. The inhaler device of any preceding claim, wherein the inhaler device is a metered dose inhaler, a dry powder inhaler, a breath actuated inhaler or a soft mist inhaler, and/or wherein the electronic component is provided within a water resistant or waterproof compartment.

11. The inhaler device of any preceding claim, wherein the electronic component comprises a camera or other imagining sensor to image a portion of the users mouth and/or throat.

12. The inhaler device of any preceding claim, wherein the electronic component comprises a microphone operable to record or otherwise monitor sound produced when the user inhales and/or exhales, to assist in gauging respiratory function.

13. An inhaler system (10), the system comprising:
an inhaler device (12) according to any one of claims 1-12, and
a computer programme application (14) operable, when run on a client device (16), to cause the client device to:
receive inhaler data from the electronic component.

14. The inhaler system of claim 13, wherein:
the computer programme application (14) is further operable to receive supplementary data from a data source other than the electronic component, wherein the supplementary data comprises at least one of: user data, sensor data, and environment data; and/or the computer programme application is further operable to transmit the received inhaler data and supplementary data to a remote server (30) over a network (26); and/or the computer
programme application is further operable to store the received inhaler data and supplementary data in a memory (28) of the client device;and/or
(i) the inhaler data comprises location data indicating a physical location of the inhaler device, and the computer programme application is further operable to display to a user of the client device a current location of the inhaler device; and/or
(ii) the inhaler data comprises usage data indicating one or more times at which a dose was administered using the inhaler device, or that zero doses have been administered using the inhaler device, and the computer programme application is further operable to display the usage data to a user of the client device; and/or
(iii) the inhaler data comprises dosage data indicating a number of doses administered using the inhaler device, and the computer programme application is further operable to display the dosage data to a user of the client device; and/or the computer programme application is in communication with network, and the computer programme application is operable to search for and retrieve environment data using the network; and/or the computer programme application is in communication with network, and the computer programme application is operable to receive anonymised inhaler data and/or anonymised supplementary data from other users of the system.

## Patentansprüche

1. Inhalationsvorrichtung (12) zur Abgabe einer Dosis eines inhalierbaren Wirkstoffs, wobei die
Inhalationsvorrichtung umfasst:
ein Gehäuse (36) mit einem Mundstück (38);
einen entfernbaren Durchflussregler (50), der zumindest teilweise in dem Gehäuse aufgenommen ist, wobei der Durchflussregler einen Hohlraum (52) definiert, der dazu dient, einen Behälter (54) aufzunehmen, der den Wirkstoff enthält; und **gekennzeichnet durch**
eine elektronische Komponente (18), die dazu dient, Inhalationsdaten zu überwachen und die Inhalationsdaten an ein entferntes Gerät (24, 30) zu übertragen, wobei sich die elektronische Komponente zusammen mit dem abnehmbaren Durchflussregler so befindet, dass der abnehmbare Durchflussregler und die elektronische Komponente zusammen einen abnehmbaren Kern (56) bilden.

2. Die Inhalationsvorrichtung nach Anspruch 1, wobei die elektronische Komponente eine Stromquelle umfasst.

3. Die Inhalationsvorrichtung nach einem der vorstehenden Ansprüche, wobei der abnehmbare Durchflussregler ein abnehmbarer Filter ist.

4. Die Inhalationsvorrichtung nach einem der vorstehenden Ansprüche, wobei die Inhalationsvorrichtung ferner eine Kappe (74) umfasst, die zum Abdecken des Mundstücks betätigt werden kann, wobei die Kappe optional mit dem Gehäuse und/oder dem Durchflussregler unter Verwendung eines Magnetverschlusses befestigt ist und wobei Daten und/oder Strom optional über den Magnetverschluss zu oder von der elektronischen Komponente übertragen werden.

5. Die Inhalationsvorrichtung nach einem der vorstehenden Ansprüche, wobei die Inhalationsdaten eines oder mehrere der folgenden Elemente umfassen: Nutzungsdaten, Standortdaten und Dosierungsdaten.

6. Die Inhalationsvorrichtung nach einem der vorstehenden Ansprüche, wobei die Inhalationsvorrichtung einen Dosierzähler (64) umfasst, der betätigt werden kann, um eine Anzahl von durch die Inhalationsvorrichtung verabreichten Dosen zu zählen, wobei optional die elektronische Komponente betätigt werden kann, um eine Warnung zu übertragen, wenn die Anzahl der verabreichten Dosen einen vordefinierten Schwellenwert überschreitet oder eine definierte Anzahl nicht erreicht.

7. Das Inhalationsgerät nach einem der vorstehenden Ansprüche, das ferner eine Anzeigekomponente
(70) umfasst, wobei die Anzeigekomponente optional ein Anzeigering ist, der zwischen dem Gehäuse und dem abnehmbaren Durchflussregler angeschlossen werden kann, und/oder wobei der abnehmbare Durchflussregler eine Struktur (51, 53) umfasst, die betätigt werden kann, um eine Geschwindigkeit eines durch die Inhalationsvorrichtung strömenden Fluids zu regulieren, wobei die Struktur optional oder vorzugsweise einen oder mehrere Kanäle, Vertiefungen oder Öffnungen umfasst.

8. Die Inhalationsvorrichtung nach einem der vorstehenden Ansprüche, wobei das Gehäuse einen Körperabschnitt
(40) mit einer ersten Längsachse definiert und ein Mundstückabschnitt (42) mit einer zweiten Längsachse in einem Winkel zur ersten Längsachse ausgerichtet ist, wobei der Winkel im Bereich von 130 bis 135 Grad liegt, und/oder wobei:
(i) das Gehäuse einen durchscheinenden Abschnitt umfasst oder im Wesentlichen durchscheinend ist; und/oder
(ii) das Gehäuse einen Metallabschnitt und/oder einen undurchsichtigen farbigen abschnitt umfasst.

9. Die Inhalationsvorrichtung nach einem der vorstehenden Ansprüche, wobei eine Außenfläche (62) des Durchflussreglers im Wesentlichen an einer Innenwand (58) des Gehäuses anliegt, so dass, wenn der Behälter in den Hohlraum innerhalb des Durchflussreglers eingeführt wird, der Durchflussregler im Wesentlichen einen Raum zwischen dem Behälter und der Innenwand des Gehäuses ausfüllt.

10. Die Inhalationsvorrichtung nach einem der vorstehenden Ansprüche, wobei die Inhalationsvorrichtung ein Dosierinhalator, ein Trockenpulverinhalator, ein atemgesteuerter Inhalator oder ein Soft-Mist-Inhalator ist und/oder wobei die elektronische Komponente in einem wasserfesten oder wasserdichten Fach untergebracht ist.

11. Inhalationsgerät nach einem der vorstehenden Ansprüche, wobei die elektronische Komponente eine Kamera oder einen anderen Bildsensor umfasst, um einen Teil des Mundes und/oder Rachens des Benutzers abzubilden.

12. Die Inhalationsvorrichtung nach einem der vorstehenden Ansprüche, wobei die elektronische Komponente umfasst
ein Mikrofon umfasst, das betätigt werden kann, um Geräusche aufzuzeichnen oder anderweitig zu überwachen, die beim Ein- und/oder Ausatmen des Benutzers entstehen, um die Messung der Atemfunktion zu unterstützen.

13. Ein Inhalationssystem (10), wobei das System umfasst:
eine Inhalationsvorrichtung (12) gemäß einem der Ansprüche 1 bis 12 und
eine Computeranwendung (14), die, wenn sie auf einem Clientgerät (16) ausgeführt wird, das Clientgerät dazu veranlasst
Inhalationsdaten von der elektronischen Komponente empfängt.

14. Das Inhalationssystem nach Anspruch 13, wobei:
die Computerprogramm-Anwendung (14) ferner so betrieben werden kann, dass sie Zusatzdaten von einer anderen Datenquelle als der elektronischen Komponente empfängt, wobei die Zusatzdaten mindestens eines der folgenden Elemente umfassen: Benutzerdaten, Sensordaten und Umgebungsdaten; und/oder die Computerprogramm-Anwendung ferner so betrieben werden kann, dass sie die empfangenen Inhalatordaten und Zusatzdaten über ein Netzwerk (26) an einen Remote-Server (30) überträgt; und/oder die Computerprogramm-Anwendung ferner so betrieben werden kann, dass sie die empfangenen Inhalator-Daten und Zusatzdaten in einem Speicher (28) des Client-Geräts speichert; und/oder
(i) die Inhalator-Daten Standortdaten umfassen, die einen physischen Standort des Inhalatorgeräts angeben, und die Computerprogramm-Anwendung ferner so betrieben werden kann, dass sie einem Benutzer des Client-Geräts einen aktuellen Standort des Inhalatorgeräts anzeigt; und/oder
(ii) die Inhalator-Daten Nutzungsdaten umfassen, die einen oder mehrere Zeitpunkte angeben, zu denen eine Dosis unter Verwendung des Inhalatorgeräts verabreicht wurde, oder dass unter Verwendung des Inhalatorgeräts keine Dosen verabreicht wurden, und die Computerprogramm-Anwendung ferner so betrieben werden kann, dass sie die Nutzungsdaten einem Benutzer des Client-Geräts anzeigt; und/oder
(iii) die Inhalator-Daten Dosierungsdaten umfassen, die eine Anzahl von Dosen angeben, die unter Verwendung des Inhalatorgeräts verabreicht wurden, und die Computerprogramm-Anwendung ferner so betrieben werden kann, dass sie die Dosierungsdaten einem Benutzer des Client-Geräts anzeigt; und/oder die Computerprogramm-Anwendung mit einem Netzwerk in Verbindung steht und die Computerprogramm-Anwendung so betrieben werden kann, dass sie unter Verwendung des Netzwerks nach Umgebungsdaten sucht und diese abruft; und/oder die Computerprogramm-Anwendung steht in Verbindung mit einem Netzwerk, und die Computerprogramm-Anwendung ist in der Lage, anonymisierte Inhalator-Daten und/oder anonymisierte Zusatzdaten von anderen Benutzern des Systems zu empfangen.

## Revendications

1. Dispositif inhalateur (12) pouvant être utilisé pour administrer une dose d'un ingrédient actif inhalable, le
inhalateur comprenant :
un boîtier (36) comprenant un embout buccal (38) ;
un régulateur de débit amovible (50) au moins partiellement logé dans le boîtier, le régulateur de débit définissant une cavité (52) pouvant recevoir un récipient (54) contenant l'ingrédient actif ; et **caractérisé par**
un composant électronique (18) pouvant être actionné pour surveiller les données de l'inhalateur et pour transmettre les données de l'inhalateur à un dispositif distant (24, 30), dans lequel le composant électronique est situé avec le régulateur de débit amovible de telle sorte que, ensemble, le régulateur de débit amovible et le composant électronique forment un noyau amovible (56).

2. Le dispositif d'inhalation selon la revendication 1, dans lequel le composant électronique comprend une source d'alimentation.

3. Le dispositif d'inhalation de l'une quelconque des revendications précédentes, dans lequel le régulateur de débit amovible est un filtre amovible.

4. Dispositif inhalateur selon l'une quelconque des revendications précédentes, dans lequel le dispositif inhalateur comprend en outre un capuchon (74) pouvant être actionné pour recouvrir l'embout buccal, dans lequel le capuchon est éventuellement fixé au boîtier et/ou au régulateur de débit à l'aide d'une fermeture magnétique et dans lequel les données et/ou l'alimentation sont éventuellement transférées vers ou depuis le composant électronique via la fermeture magnétique.

5. Dispositif d'inhalation selon l'une quelconque des revendications précédentes, dans lequel les données d'inhalation comprennent une ou plusieurs des données suivantes : données d'utilisation, données de localisation et données de dosage.

6. Dispositif d'inhalation selon l'une quelconque des revendications précédentes, dans lequel le dispositif d'inhalation comprend un compteur de dosage (64) pouvant être actionné pour compter un nombre de doses administrées par le dispositif d'inhalation, dans lequel, en option, le composant électronique peut être actionné pour transmettre une alerte lorsque le nombre de doses administrées dépasse un seuil prédéfini ou n'atteint pas un nombre défini.

7. Le dispositif inhalateur de l'une quelconque des revendications précédentes, comprenant en outre un composant indicateur
(70), dans lequel le composant indicateur est éventuellement une bague indicatrice pouvant être connectée entre le boîtier et le régulateur de débit amovible, et/ou dans lequel le régulateur de débit amovible comprend une structure (51, 53) pouvant être actionnée pour réguler la vitesse d'un fluide s'écoulant à travers le dispositif d'inhalation, dans lequel la structure comprend éventuellement ou de préférence un ou plusieurs canaux, alvéoles ou ouvertures.

8. Le dispositif d'inhalation de l'une quelconque des revendications précédentes, dans lequel le boîtier définit une partie de corps
(40) ayant un premier axe longitudinal et une partie d'embouchure (42) ayant un deuxième axe longitudinal orienté selon un angle par rapport au premier axe longitudinal, dans lequel l'angle est compris entre 130 et 135 degrés, et/ou dans lequel :
(i) le boîtier comprend une partie translucide ou est sensiblement translucide ; et/ou
(ii) le boîtier comprend une partie métallique et/ou une partie colorée opaque.

9. Dispositif inhalateur selon l'une quelconque des revendications précédentes, dans lequel une surface extérieure (62) du régulateur de débit vient sensiblement en butée contre une paroi intérieure (58) du boîtier, de telle sorte que lorsque le récipient est inséré dans la cavité à l'intérieur du régulateur de débit, le régulateur de débit remplit sensiblement un espace entre le récipient et la paroi intérieure du boîtier.

10. Dispositif d'inhalation selon l'une quelconque des revendications précédentes, dans lequel le dispositif d'inhalation est un inhalateur-doseur, un inhalateur à poudre sèche, un inhalateur actionné par la respiration ou un inhalateur à vapeur douce, et/ou dans lequel le composant électronique est prévu à l'intérieur d'un compartiment résistant à l'eau ou étanche.

11. Le dispositif d'inhalation de l'une quelconque des revendications précédentes, dans lequel le composant électronique comprend une caméra ou un autre capteur d'imagerie pour former une image d'une partie de la bouche et/ou de la gorge de l'utilisateur.

12. Dispositif inhalateur selon l'une quelconque des revendications précédentes, dans lequel le composant électronique comprend
un microphone pouvant être utilisé pour enregistrer ou surveiller de toute autre manière le son produit lorsque l'utilisateur inspire et/ou expire, afin d'aider à évaluer la fonction respiratoire.

13. Système d'inhalation (10), le système comprenant :
un dispositif d'inhalation (12) selon l'une quelconque des revendications 1 à 12, et
une application informatique (14) pouvant être utilisée, lorsqu'elle est exécutée sur un dispositif client (16), pour amener le dispositif client à :
reçoive des données d'inhalateur provenant du composant électronique.

14. Le système d'inhalateur selon la revendication 13, dans lequel :
l'application informatique (14) est en outre capable de recevoir des données supplémentaires provenant d'une source de données autre que le composant électronique, les données supplémentaires comprenant au moins l'une des données suivantes : données utilisateur, données de capteur et données environnementales ; et/ou l'application informatique est en outre capable de transmettre les données d'inhalateur et les données supplémentaires reçues à un serveur distant (30) via un réseau (26) ; et/ou l'application informatique est en outre capable de stocker les données d'inhalateur et les données supplémentaires reçues dans une mémoire (28) du dispositif client ; et/ou
(i) les données de l'inhalateur comprennent des données de localisation indiquant l'emplacement physique du dispositif inhalateur, et l'application informatique est en outre capable d'afficher à un utilisateur du dispositif client l'emplacement actuel du dispositif inhalateur ; et/ou
(ii) les données relatives à l'inhalateur comprennent des données d'utilisation indiquant une ou plusieurs fois où une dose a été administrée à l'aide du dispositif inhalateur, ou qu'aucune dose n'a été administrée à l'aide du dispositif inhalateur, et l'application informatique est en outre capable d'afficher les données d'utilisation à un utilisateur du dispositif client ; et/ou
(iii) les données relatives à l'inhalateur comprennent des données de dosage indiquant un nombre de doses administrées à l'aide du dispositif inhalateur, et l'application informatique est en outre capable d'afficher les données de dosage à un utilisateur du dispositif client ; et/ou l'application informatique est en communication avec un réseau, et l'application informatique est capable de rechercher et de récupérer des données environnementales à l'aide du réseau ; et/ou l'application informatique est en communication avec le réseau, et l'application informatique est capable de recevoir des données anonymisées relatives à l'inhalateur et/ou des données supplémentaires anonymisées provenant d'autres utilisateurs du système.
